# FASCICULE DE BREVET EUROPEEN

(11) **EP 0 850 643 B1**
(45) Date de publication et mention de la délivrance du brevet: **15.12.1999**
(21) Numéro de dépôt: 97403029.8
(22) Date de dépôt: 12.12.1997
(51) Int. Cl.: A61K 7/48, A61K 7/02, A61K 7/021, A61K 7/027

(54) **Composition de maquillage ou de soin, sans transfert, contenant un organopolysiloxane solide élastomère et une phase grasse**
Zusammensetzung zum Schminken oder zur Pflege ohne Transfer, die ein elastomeres, festes Organopolysiloxan und eine Fettphase enthält
Make up or personal care composition which does not rub off and which comprises a solid elastomeric organopolysiloxane and a fatty phase

(30) Priorité: 24.12.1996 FR 9615984
(43) Date de publication de la demande: 01.07.1998
(73) Titulaire: L'OREAL, 75008 Paris (FR)
(72) Inventeur: Bara, Isabelle, 75013 Paris (FR); Auguste, Frédéric, 94550 Chevilly Larue (FR)
(74) Mandataire: Lhoste, Catherine

(56) Documents cités:
- CHEMICAL ABSTRACTS, vol. 126, no. 8, 24 février 1997 Columbus, Ohio, US; abstract no. 108665, KURODA, AKIHIRO: "Makeup cosmetics containing fine powders and fluoroalkyl-modified silicates" XP002042884 & JP 08 301 727 A (KANEBO LTD, JAPAN) 19 novembre 1996
- CHEMICAL ABSTRACTS, vol. 126, no. 8, 24 février 1997 Columbus, Ohio, US; abstract no. 108664, KURODA, AKIHIRO ET AL: "Sunscreens containing metal oxides, polyoxyalkylene-polysiloxanes, and elastomers or resin waxes" XP002042885 & JP 08 295 620 A (KANEBO LTD, JAPAN) 12 novembre 1996
- CHEMICAL ABSTRACTS, vol. 126, no. 9, 3 mars 1997 Columbus, Ohio, US; abstract no. 122311, YANAGIDA, TAKESHI ET AL: "Manufacture of cosmetic makeups with organopolysiloxane elastomers for covering pores of hairs" XP002042886 & JP 08 319 215 A (SHISEIDO CO LTD, JAPAN) 3 décembre 1996
- CHEMICAL ABSTRACTS, vol. 124, no. 4, 22 janvier 1996 Columbus, Ohio, US; abstract no. 37399, TAKAHASHI, HIDEKI ET AL: "Two-layer oily makeup cosmetics containing spherical powders of organopolysiloxane elastomers and hydrophobic-treated powders" XP002042887 & JP 07 267 820 A (SHISEIDO CO LTD, JAPAN) 17 octobre 1995
- CHEMICAL ABSTRACTS, vol. 110, no. 14, 3 avril 1989 Columbus, Ohio, US; abstract no. 121003, PROCTER AND GAMBLE CO., USA: "Hair conditioners containing silicone polymers and volatile carriers" XP002042888 & JP 63 022 010 A (PROCTER AND GAMBLE CO., USA) 29 janvier 1988
- CHEMICAL ABSTRACTS, vol. 127, no. 3, 21 juillet 1997 Columbus, Ohio, US; abstract no. 39538, KURODA, AKIHIRO: "Cosmetics containing silicon-containing resins" XP002060246 & JP 09 143 029 A (KANEBO, LTD., JAPAN) 3 juin 1997
- CHEMICAL ABSTRACTS, vol. 127, no. 12, 22 septembre 1997 Columbus, Ohio, US; abstract no. 166546, YANAGIDA, TAKESHI ET AL: "Makeups containing powders of silicone rubber and water-absorbing polymer" XP002060247 & JP 09 175 939 A (SHISEIDO CO., LTD., JAPAN) 8 juillet 1997
- CHEMICAL ABSTRACTS, vol. 127, no. 12, 22 septembre 1997 Columbus, Ohio, US; abstract no. 166545, YANAGIDA, TAKESHI ET AL: "Makeups containing silicone rubber and silicone oils and resins" XP002060248 & JP 09 175 940 A (SHISEIDO CO., LTD., JAPAN) 8 juillet 1997

## Description

La présente invention se rapporte à l'utilisation d'un organopoysiloxane dans une composition de soin et/ou de maquillage de la peau et/ou des lèvres des êtres humains, et en particulier à un rouge à lèvres ou un fond de teint, sous forme de stick, de coupelle ou de crème.

Les compositions de rouge à lèvres et de fond de teint connues comprennent généralement des corps gras tels que des huiles, des composés pâteux et des cires, ainsi qu'une phase particulaire généralement composée de charges et de pigments.

Ces compositions, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact, et notamment un verre, une tasse, une cigarette, un vêtement ou la peau. Il s'ensuit une persistance médiocre du film appliqué sur la peau ou les lèvres nécessitant de renouveler régulièrement l'application de la composition de fond de teint ou de rouge à lèvres. Par ailleurs, l'apparition de ces traces inacceptables notamment sur les cols de chemisier peut écarter certaines femmes à utiliser ce type de maquillage.

Lié à cet inconvénient de transfert des compositions de rouge à lèvres de l'art antérieur, il faut encore noter la fâcheuse tendance aux films de ces compositions à se dissoudre dans les huiles végétales généralement utilisées dans certains plats cuisinés comme les salades à la vinaigrette, obligeant les femmes à aller se remaquiller les lèvres après le repas.

Depuis plusieurs années, les cosméticiens se sont intéressés aux compositions de rouge à lèvres et plus récemment aux compositions de fond de teint 〈〈 sans transfert 〉〉. Ainsi, la société Shiseido a envisagé dans sa demande de brevet JP-A-61-65809 des compositions de rouge à lèvres 〈〈 sans transfert 〉〉 contenant de 1 à 70% en poids d'une résine siloxysilicate (à réseau tridimensionnel) comportant des chaînes pendantes alkylées de 1 à 6 atomes de carbone ou phénylées, de 10 à 98% en poids d'une huile de silicone volatile à chaîne silicone cyclique et des charges pulvérulentes. De même la société Noevier à décrit dans le document JP-A-62-61911 des compositions de rouge à lèvres, d'eye liner, de fonds de teint 〈〈 sans transfert 〉〉 comportant une ou plusieurs silicones volatiles associées à une ou plusieurs cires hycrocarbonées.

Ces compositions, bien que tout-à-fait satisfaisantes pour la propriété de 〈〈 sans transfert 〉〉 présentent l'inconvénient de laisser sur les lèvres, après évaporation des huiles de silicone, un film qui devient inconfortable au cours du temps (sensation de dessèchement et de tiraillement), écartant un certain nombre de femmes de ce type de rouge à lèvres. Pour améliorer le confort de ce type de composition, on pourrait ajouter des huiles non volatiles siliconées ou non, mais dans ce cas on perd en efficacité 〈〈 sans transfert 〉〉.

Plus récemment, la société Revlon a envisagé dans sa demande de brevet EP-A-602905 des rouges à lèvres 〈〈 sans transfert 〉〉 contenant une silicone volatile cyclique ou linéaire à chaînes méthyle pendantes et une résine de silicone comportant une chaîne estérifiée pendante ayant de 12 à 18 atomes de carbone. Le film de rouge à lèvres, restant sur les lèvres après évaporation de la silicone volatile, a encore l'inconvénient de manquer de confort à l'application et notamment d'être trop sec. Elle a, en outre, envisagé dans sa demande de brevet EP-A-709 083 des fonds de teint 〈〈 sans transfert 〉〉 contenant une silicone volatile et associée à une résine siloxysilicate. Ces fonds de teint présentent encore l'inconvénient d'être peu confortables et secs au cours du temps.

La présente invention a justement pour objet l'utilisation d'organosiloxane particulier dans une composition de soin ou de maquillage permettant de remédier à ces inconvénients et permettant en particulier l'obtention d'un film ne transférant pas et présentant des propriétés cosmétiques améliorées par rapport à celles des produits 〈〈 sans transfert 〉〉 de l'art antérieur, notamment des propriétés de glissant, de non tiraillement et de non dessèchement des lèvres.

L'invention s'applique non seulement aux produits de maquillage des lèvres mais aussi aux produits de soin et/ou de traitement des lèvres, ainsi qu'aux produits de maquillage et de soin de la peau.

Ainsi, l'invention a pour objet l'utilisation d'au moins un organopolysiloxane au moins partiellement réticulé solide élastomérique associé à une phase grasse renfermant au moins une huile volatile a température ambiante dans une composition de maquillage ou de soin sans transfert.

Par 〈〈 élastomérique 〉〉 on entend un matériau souple, déformable ayant des propriétés viscoélastiques et notamment la consistance d'une éponge ou d'une sphère souple.

Les organopolysiloxanes élastomériques de la composition de l'invention présentent un remarquable pouvoir gélifiant d'huile. Ils ne sont pas desséchants pour la peau et apportent de bonnes propriétés cosmétiques. Ces nouveaux élastomères conduisent à des compositions confortables à l'application, douces et non collantes au toucher. Cette douceur est due, d'une part à la texture des organopolysiloxanes et, d'autre part à leurs propriétés comparables à celles de microéponges piégeant les notamment les huiles non volatiles. Les organopolysiloxanes selon l'invention, en piégeant les huiles non volatiles assurent, en outre, les propriétés de sans transfert de la composition.

Ces compositions après évaporation de l'huile volatile conduisent à un film homogène et uniforme ayant une texture légère il est confortable, non sec, ne tiraille pas et peut être porté tout au long de la journée.

La composition de l'invention peut se présenter sous forme de pâte, de solide, de crème. Elle peut être une émulsion huile-dans-eau ou eau-dans-huile, un gel anhydre, solide ou souple.

Les organopolysiloxanes élastomériques conformes à l'invention sont partiellement ou totalement réticulés et de structure tridimensionnelle. Inclus dans une phase grasse, ils se transforment, selon le taux de phase grasse utilisé, d'un produit d'aspect spongieux lorsqu'ils sont utilisés en présence de faibles teneurs en phase grasse en un gel homogène, en présence de quantités de phase grasse plus élevées. La gélification de la phase grasse par ces élastomères peut être totale ou partielle.

Les élastomères de l'invention se présentent généralement sous forme de gel constitué d'un organopolysiloxane élastomère de structure tridimensionnelle, inclus dans au moins une huile hydrocarbonée et/ou une huile siliconée.

Les organopolysiloxanes élastomériques selon l'invention peuvent être choisis parmi les polymères réticulés décrits dans la demande EP-A-0295886. Selon cette demande, ils sont obtenus par réaction d'addition et de réticulation, en présence d'un catalyseur du type platine, d'au moins :
- (a) un organopolysiloxane ayant au moins deux groupes alcényle inférieurs en C₂-C₆ par molécule ; et
- (b) un organopolysiloxane ayant au moins deux atomes d'hydrogène liés à un atome de silicium par molécule.

Les organopolysiloxanes élastomériques selon l'invention peuvent aussi être choisis parmi ceux décrits dans le brevet US 5.266.321. Selon ce brevet, ils sont choisis notamment parmi :
- i) les organopolysiloxanes comprenant des motifs R₂SiO et RSiO_{1,5} et éventuellement des motifs R₃SiO_{0,5} et/ou SiO₂ dans lesquels les radicaux R, indépendamment les uns des autres, représentent un hydrogène, un alkyle tel que méthyle, éthyle ou propyle, un aryle tel que phényle ou tolyle, un groupe aliphatique insaturé tel que vinyle, le rapport en poids des motifs R₂SiO sur les motifs RSiO_{1,5} allant de 1/1 à 30/1 ;
- ii) les organopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol lorsque l'organopolysiloxane est non-cyclique et entre 1 et 50% mol lorsque l'organopolysiloxane est cyclique.

Les organopolysiloxanes de la composition de l'invention sont par exemple ceux commercialisés sous les noms KSG6 de Shin-Etsu, Trefil E-505C ou Trefil E-506C de Dow-Corning, Gransil de Grant Industries (SR-CYC, SR DMF10, SR-DC556), ou ceux commercialisés sous forme de gels déjà constitués (KSG15, KSG17, KSG16, KSG18 de Shin-Etsu, Gransil SR 5CYC gel, Gransil SR DMF 10 gel, Gransil SR DC556 gel, SF 1204 et JK 113 de General Electric. On peut aussi utiliser un mélange de ces produits commerciaux.

De façon préférentielle, l'organopolysiloxane est présent dans le mélange organo-polysiloxane/phase grasse sous forme d'un gel homogène à une concentration allant de 0,3 à 40 % du poids total de la composition. De préférence l'élastomère représente, en matière active, de 0,1 à 20% dans la composition et de préférence de 0,3 à 15%.

La phase grasse peut être quelconque ; elle contient généralement des huiles (produits fluides à température ambiante) qui peuvent êtres des huiles siliconées, fluorées, fluorosiliconées, hydrocarbonées éventuellement partiellement siliconées. La meilleure gélification est réalisée avec les huiles siliconées ou partiellement siliconées, des huiles apolaires et peu polaires et quelques huiles polaires qui ne nuisent pas à la stabilité du système.

Selon l'invention, la phase grasse contient une ou plusieurs huiles volatiles à température ambiante. Par huile volatile, on entend une huile susceptible de s'évaporer au contact de la peau ou des lèvres.

Ces huiles volatiles peuvent être des huiles hydrocarbonées, des huiles de silicones ou leurs mélanges. Les silicones volatiles sont par exemple des silicones comportant une structure silicone linéaire et des motifs à chaîne alkyle pendante et'ou en bout de structure silicone, ces chaînes alkyle étant linéaires ou ramifiées et comportant de 3 à 10 atomes de carbone. Les silicones volatiles à chaîne alkyle présentent notamment la formule (1) suivante :

R'(CH₃)₂Si-O-{-Si-(CH₃)R''-O-}ₙ-{-Si-(CH₃)₂-O-}ₘ-Si(CH₃)₂R'

où R' et R'' sont indépendamment H, méthyle ou une chaîne ayant de 3 à 10 atomes de carbone, n et m étant des entiers allant de 0 à 10 à condition que si R' est hydrogène ou méthyle, n est différent de 0 et R'' représente une chaîne alkyle de3 à 10 atomes de carbone. Comme silicones volatiles alkylées utilisables dans l'invention, on peut citer les alkyl heptaméthyltrisiloxanes avec un groupe alkyle en C₄, C₅, C₆, C₇ et C₈ comme par exemple l'hexyl heptaméthyltrisiloxane de formule : (CH₃)₃-Si-O-Si(CH₃)(C₆H₁₃)-O-Si(CH₃)₃ ; l'octyl heptaméthyltrisiloxane de formule : (CH₃)₃-Si-O-Si(CH₃)(C₈H₁₅)-O-Si(CH₃)₃; et leurs mélanges.

Comme silicones volatiles utilisables dans l'invention, on peut encore citer les polydiméthylsiloxanes à chaîne linéaire ayant de 2 à 6 atomes de silicium. Ces silicones satisfont à la formule (1) avec m valant 0, n valant de 0 à 6 et R₁ et R₂ représentant simultanément CH₃ ou phényle. On peut par exemple citer les méthylpolysiloxanes comme l'hexaméthyldisiloxane, les méthylphénylpolysiloxanes, les éthylpolysiloxanes, les éthylméthylpolysiloxanes, les éthylphénylpolysiloxanes, les hydroxyméthylpolysiloxanes et leurs mélanges.

Comme silicones volatiles, on peut aussi utiliser des silicones cycliques ayant de 3 à 7 motifs -R₁R₂SiO-, avec R₁ et R₂ représentant indépendamment H, méthyle, éthyle ou phényle. A titre d'exemple, on peut citer l'octamèthylcyclopentasiloxane, le décaméthylcyclùpentasiloxane ou leurs mélanges.

Comme huiles volatiles hydrocarbonées utilisables dans l'invention, on peut citer les isoparaffines en C₃ à C₂₀ comme l'isoparaffine en C₁₂ appelé isododécane et l'isoparaffine en C₁₆ comme l'isohexadécane.

Les huiles volatiles représentent avantageusement de 1 à 50% du poids total de la composition et mieux de 30 à 50%.

La composition de l'invention peut comprendre avantageusement, en plus des huiles volatiles mentionnées ci-dessus, des corps gras non volatiles usuellement utilisés dans le domaine d'application envisagé et notamment des huiles non volatiles et des cires. La présence de ces huiles non volatiles améliore le confort de la composition.

Commes huiles non volatiles utilisables dans l'invention, on peut citer notamment :
- les huiles hydrocarbonées d'origine animale telles que le perhydrosqualène ;
- les huiles hydrocarbonées végétales telles que les triglycérides liquides d'acides gras, par exemple les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel ;
- les huiles de formule R₉COOR₁₀ dans laquelle R₉ représente le reste d'un acide gras supérieur comportant de 7 à 19 atomes de carbone et R₁₀ représente une chaîne hydrocarbonée ramifiée contenant de 3 à 20 atomes de carbone comme par exemple l'huile de Purcellin ;
- les hydrocarbures linéaires ou ramifiés, d'origine minérale ou synthétique tels que les huiles de paraffine non volatiles et leurs dérivés, la vaseline, les polydécènes, le polyisobutène hydrogéné tel que le parléam ;
- les esters et les éthers de synthèse comme le myristate d'isopropyle, des octanoates, décanoates ou ricinoléates d'alcools ou de polyalcools ;
- des alcools gras comme l'octyl dodécanol ou l'alcool oléique ;
- les huiles fluorées partiellement hydrocarbonées et/ou siliconées comme celle décrites dans le document JP-A-2-295912 ;
- les huiles siliconées comme les polyméthylsiloxanes non volatiles linéaires, liquides ou pâteux à température ambiante, les phényl diméthicones, les phényl triméthicones et les polyméthylphénylsiloxanes ;
- leurs mélanges.

Les huiles non volatiles représentent de 0 à 50% du poids total de la composition, de préférence entre 0 et 35% et sont choisies en fonction de leur compatibilité avec les organopolysiloxanes élastomériques.

Avantageusement, la composition selon l'invention peut contenir des cires hydrocarbonées, fluorées ou siliconées ou leurs mélanges, qui peuvent être solides ou semi-solides (sous forme d'une pâte) à température ambiante. Ces cires peuvent être d'origine végétale, minérale, animale et'ou synthétique. En particulier, ces cires présentes une température de fusion supérieure à 25 °C et mieux supérieure à 45°C.

Les cires siliconées peuvent être des cires comportant une structure siliconée et des motifs à une ou plusieurs chaînes alkyle ou alcoxy pendantes et/ou en bout de structure siliconée, ces chaînes étant linéaires ou ramifiées et comportant de 10 à 45 atomes de carbone. Ces cires sont appelées respectivement des alkyldi10 à 45 atomes de carbone. Ces cires sont appelées respectivement des alkyldiméthicones et des alcoxydiméthicones. Par ailleurs, ces chaînes alkyle peuvent comporter une ou plusieurs fonctions ester.

Parmi les cires de silicone utilisables dans l'invention, on peut citer la béhénoxy diméthicone telle que celle vendue par Goldschmidt sous le nom Abil Wax 2440 ; la stéaryl diméthicone telle que celle vendue par Dow Corning sous le nom DC 2503 ; la cétyl diméthicone telle que celle vendue par Goldschmidt sous le nom Abil Wax 9814 ; la stéaryl méthicone telle que celle vendue par Goldschmidt sous le nom Abil Wax 9809 ; la C₂₄-C₂₈ alkyl diméthicone telle que celle vendue par Goldschmidt sous le nom Abil Wax 9810 ; la C₃₀-C₄₅ alkyl méthicone telle que celle vendue par Goldschmidt sous le nom Abil Wax 9811 ; la stéaroxy diméthicone telle que celle vendue par Goldschmidt sous le nom Abil Wax 2434 ; la béhénate diméthicone comme celle vendue par Rhône Poulenc sous le nom Myrasil Wax B.

Comme autres cires de silicone utilisables dans l'invention, on peut citer les copolymères d'alkyl diméthicones. Ces copolymères sont notamment ceux décrits dans les documents EP-A-527594, US-A-5 061 481, US-A- 5397 566, EP-A-527594. On peut aussi citer les cires de silicones modifiées par des chaînes fluorées comme celles décrites dans le document EP-A-661 042.

Comme autre cires utilisables dans l'invention, on peut citer les cires d'origine animale comme la lanoline, la cire d'abeilles ; les cires végétales telles que la cire de Carnauba ou de Candellila ; les cires minérales, par exemple de paraffine, de lignite ou les cires microcristallines, la cérésine ou l'ozokérite ; les cires synthétiques comme les cires de polyéthylène.

Ces corps gras peuvent être choisis de manière variée par l'homme du métier afin de préparer une composition ayant les propriétés, par exemple de consistance ou de texture, souhaitées.

En particulier, la présence de cires permet d'assurer une bonne résistance mécanique, notamment lorsque la composition se présente sous la forme d'un stick.

D'une manière générale, la composition peut comprendre de 0 à 50% du poids total de la composition de cire et de préférence de 10 à 30%.

La composition de l'invention peut comprendre, en outre, tout additif usuellement utilisé dans le domaine concerné, tel que des colorants hydrosolubles ou liposolubles, des antioxydants, des huiles essentielles, des conservateurs, des actifs cosmétiques ou dermatologiques, des hydratants, des vitamines, des acides gras essentiels, des filtres solaires lipophiles, des polymères liposolubles notamment hydrocarbonés tels que les polyalkylènes. Ces additifs peuvent être présents dans la composition à raison de 0 à 20% du poids total de la composition et mieux de 0 à 10%.

Bien entendu l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée. En particulier, ces additifs ne devront pas nuire à l'homogénéité, la stabilité, le confort et au 〈〈 non transfert 〉〉 de la composition.

Les compositions selon l'invention peuvent se présenter notamment sous la forme de stick ou bâton, ou sous la forme de pâte souple ou coulée, voire sous la forme d'un liquide huileux gélifié ou d'une crème.

La composition selon l'invention peut se présenter sous la forme d'un produit coloré de maquillage de la peau, en particulier un fond de teint, un fard à joues ou à paupières, ou un stick anti-cernes ou de maquillage des lèvres comme un rouge à lèvres. Elles peuvent également se présenter sous forme non colorée, contenant éventuellement des actifs cosmétiques ou dermatologiques. Elle peut alors être utilisée comme base de soin pour les lèvres (baumes à lèvres, protégeant les lèvres du froid et/ou du soleil et/ou du vent) ou base fixante à appliquer sur un rouge à lèvres classique. La base fixante forme alors un film protecteur sur le film de rouge, qui en limite le transfert.

La composition de l'invention peut également se présenter sous la forme d'une composition dermatologique ou de soin de la peau, ou sous forme d'une composition de protection solaire.

Bien entendu la composition de l'invention doit être cosmétiquement ou dermatologiquement acceptable, à savoir non toxique et susceptible d'être appliquée sur la peau ou les muqueuses (lèvres, intérieur des paupières) d'être humains.

De façon préférentielle, la composition de l'invention peut comprendre une phase particulaire, généralement présente à raison de 0 à 35 % du poids total de la composition, de préférence de 5 à 25 %, et qui peut comprendre des pigments et/ou des nacres et/ou des charges habituellement utilisés dans les compositions cosmétiques.

Par pigments, il faut comprendre des particules blanches ou colorées, minérales ou organiques, insolubles dans la cire et la silicone volatile, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent à modifier la texture de la composition ainsi que l'effet de matité/brillance.

Les pigments peuvent être présents dans la composition à raison de 0 à 25 % du poids de la composition finale, et de préférence à raison de 5 à 15 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium.

Les nacres peuvent être présentes dans la composition à raison de 0 à 20 % du poids total de la composition, de préférence à un taux élevé de l'ordre de 8 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 35 % du poids total de la composition, de préférence 5 à 15 %. On peut notamment citer le talc, le mica, la silice, le kaolin, les poudres de Nylon (Orgasol notamment) et de polyéthylène, le Téflon, l'amidon, le nitrure de bore, des microsphères de copolymères telles que l'Expancel (Nobel Industrie), le polytrap (Dow Corning) et les microbilles de résine de silicone (Tospearl de Toshiba, par exemple).

De façon plus spécifique, l'invention a pour objet un rouge à lèvres ou fond de teint anhydre sans transfert, caractérisé en ce qu'il contient au moins un organopolysiloxane au moins partiellement réticulé solide élastomérique associé à une phase grasse renfermant au moins une huile volatile à température ambiante, et des pigments et/ou des charges.

La composition selon l'invention peut être fabriquée par chauffage d'un ou plusieurs organopolysiloxanes élastomériques associés à une ou plusieurs huiles, une ou plusieurs cires, un ou plusieurs pigments, une ou plusieurs charges et/ou un ou plusieurs autres additifs à une température supérieure à la température la plus élevée de fusion des cires, puis coulage du mélange fondu dans un moule. Ce procédé permet d'obtenir une composition sous forme solide de stick ou de coupelle.

Elle peut aussi être obtenue par extrusion comme décrit dans la demande EP-A-667 146. Ce procédé consiste à malaxer la pâte (cires + huiles + additifs + pigments) pendant le refroidissement pour créer dans la masse des zones d'écrasement de la pâte à l'aide d'un broyeur à cylindres ou d'un extrudeur-mélangeur à vis. Ce procédé permet l'obtention d'une composition sous forme de pâte molle.

L'invention a encore pour objet l'utilisation de l'association d'une silicone volatile à température ambiante et d'un organopolysiloxane élastomérique solide au moins partiellement réticulé dans une composition afin de diminuer le transfert de ladite composition.

L'invention a encore pour objet un procédé pour limiter et/ou empêcher le transfert d'une composition de maquillage ou de soin de la peau ou des lèvres sur un substrat différent de la dite peau et desdites lèvres, consistant à introduire dans la composition au moins un organopolysiloxane élastomérique solide au moins partiellement réticulé et une huile volatile à température.

L'invention est illustrée plus en détail dans les exemples suivants. Les pourcentages sont donnés en poids.

### Exemple 1

| | |
|---|---|
| Phényl triméthicone | 22% |
| Stéaryl diméthicone | 4% |
| Huile de jojoba | 1% |
| Organopolysiloxane élastomérique (KSG6) | 5% |
| Pigments | 8% |
| Cire de polyéthylène | 20% |
| Cyclométhicone volatile | 40% |

*Préparation* : On fait gonfler l'orgnopolysiloxane dans les huiles non volatiles à températures ambiante. On empâte ensuite les pigments dans le gel obtenu à 60°C puis on broie le tout dans un broyeur tricylindre à température ambiante. On mélange le broyât aux cires à 95°C puis on ajoute l'huile volatile et on coule dans un moule.

On obtient un rouge à lèvres coulé, de texture agréable, qui s'étale bien et de façon homogène.

*Test* :On applique cette composition sur la partie gauche des lèvres de plusieurs personnes.

Pour comparaison, on applique sur la partie droite desdites lèvres, un rouge à lèvres 〈〈 sans transfert 〉〉 de l'art antérieur, ne comprenant pas d'organopolysiloxane élastomérique et une phase grasse.

On laisse sécher les rouges à lèvres à température ambiante pendant 30 minutes, puis on applique la totalité des lèvres sur une feuille de papier.

On constate sur la totalité des feuilles de papier une trace de rouge à lèvres très faible, à peine perceptible, aussi bien pour la composition de l'invention que pour la composition de l'art antérieur. En outre, le film obtenu sur les lèvres avec la composition de l'invention est reconnu plus confortable et moins sec que celui obtenu avec la composition de l'art antérieur.

### Exemple 2

| | |
|---|---|
| Organopolysiloxane élastomérique (KSG 6) | 10% |
| Béhénoxydiméthicone (Abil Wax 2440) | 5% |
| Cire de polyéthylène | 20% |
| Alkyldiméthicone (X2 5514 de Dow Corning) | 20% |
| Alkyldiméthicone (X2 1731 de Dow Corning) | 55% |

On prépare cette composition comme dans l'exemple 1.

Cette composition se présente sous forme de texture agréable, qui s'étale bien et s'applique uniformément. Le film obtenu sur les lèvres est confortable à porter dans le temps.

## Revendications

1. Utilisation d'un organopolysiloxane solide élastomérique au moins partiellement réticulé associé à une phase grasse contenant au moins une huile volatile à température ambiante dans une composition cosmétique ou pour la fabrication d'une composition dermatologique afin de diminuer le transfert de ladite composition.

2. Utilisation selon la revendication 1, caractérisée en ce que l'huile volatile est présente à raison de 1 à 50 % en poids, de préférence 30 à 50 %, du poids total de la composition.

3. Utilisation selon l'une des revendications précédentes, caractérisée en ce que l'huile volatile est choisie parmi les huiles de silicones comportant une structure silicone linéaire et des motifs à chaîne alkyle pendante et/ou en bout de structure silicone, ces chaînes alkyle étant linéaires ou ramifiées et comportant de 3 à 10 atomes de carbone ; les polydiméthylsiloxanes à chaîne linéaire ayant de 2 à 6 atomes de silicium ; les silicones cycliques ayant de 3 à 7 motifs -R₁R₂SiO-, avec R₁ et R₂ représentant indépendamment H, méthyle, éthyle ou phényle; les isoparaffines en C₃ à C₂₀ et leurs mélanges.

4. Utilisation selon l'une des revendications précédentes, caractérisée en ce que l'organopolysiloxane élastomérique est obtenu par réaction d'addition et de réticulation, en présence d'un catalyseur, d'au moins :
(a) un organopolysiloxane ayant au moins deux groupes alcényle inférieurs en C₂-C₆ par molécule ;
(b) un organopolysiloxane ayant au moins deux atomes d'hydrogène liés à un atome de silicium par molécule.

5. Utilisation selon l'une quelconque des revendications précédentes, caractérisée en ce que l'organopolysiloxane est choisi parmi :
- i) les organopolysiloxanes comprenant des motifs R₂SiO et RSiO_{1,5} et éventuellement des motifs R₃SiO_{0,5} et/ou SiO₂ dans lesquels les radicaux R, indépendamment les uns des autres, représentent un hydrogène, un alkyle, un aryle, un groupe aliphatique insaturé, le rapport en poids des motifs R₂SiO sur les motifs RSiO_{1,5} allant de 1/1 à 30/1 ;
- ii) les organopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol lorsque l'organopolysiloxane est non-cyclique et entre 1 et 50% mol lorsque l'organopolysiloxane est cyclique.

6. Utilisation selon l'une des revendications précédentes, caractérisée en ce que la phase grasse contient, en outre, au moins une huile non volatile.

7. Utilisation selon l'une des revendications précédentes, caractérisée en ce que l'organopolysiloxane solide élastomérique est présent dans le mélange organopolysiloxane élastomérique/phase grasse pour former un gel homogène dans une concentration allant de 0,3 à 40 % en poids.

8. Utilisation selon l'une quelconque des revendications précédentes, caractérisée en ce que la phase grasse contient, en outre, au moins une huile non volatile choisie parmi les huiles hydrocarbonées d'origine animale, végétale ; les huiles de formule R₉COOR₁₀ dans laquelle R₉ représente le reste d'un acide gras supérieur comportant de 7 à 19 atomes de carbone et R₁₀ représente une chaîne hydrocarbonée ramifiée contenant de 3 à 20 atomes de carbone; les hydrocarbures linéaires ou ramifiés d'origine minérale ou synthétique ; les alcools gras ; les esters et éthers de synthèse ; les polyméthylsiloxanes linéaires liquides ou pâteux à température ambiante ; les phényl diméthicones ; les phényl triméthicones ; les polyméthylphényl siloxanes; les huiles fluorées éventuellement partiellement hydrocarbonées et/ou siliconées ; et leurs mélanges.

9. Utilisation selon l'une quelconque des revendications précédentes, caractérisée en ce qu'elle contient, de plus, au moins une cire.

10. Utilisation selon la revendication précédente, caractérisée en ce que la cire est choisie parmi les cires hydrocarbonées, fluorées, siliconées et leurs mélanges.

11. Utilisation selon la revendication 9 ou 10, caractérisée en ce que la cire est présente à raison de 0 à 50 % du poids total de la composition, de préférence 10 à 30 %.

12. Utilisation selon l'une des revendications précédentes, caractérisée en ce que la composition comprend, en outre, une phase particulaire présente à raison de 0 à 35 % du poids total de la composition, de préférence 5 à 25 %.

13. Utilisation selon l'une des revendications précédentes, caractérisée en ce que la composition se présente sous la forme de stick ou bâton, de pâte souple ou coulée, ou d'une crème ou d'un gel.

14. Utilisation selon l'une des revendications précédentes, caractérisée en ce que la composition contient, en outre, au moins un actif cosmétique ou dermatologique.

15. Utilisation selon l'une des revendications précédentes, caractérisée en ce que la composition se présente sous forme d'une composition de fond de teint, de fard à joues ou à paupières, d'un produit anti-cernes ou de rouge à lèvres, dans une base de soin ou une base fixante pour les lèvres, d'un produit de soin de la peau ou d'une composition solaire.

16. Utilisation d'au moins un organopolysiloxane solide élastomérique au moins partiellement réticulé associé à une phase grasse renfermant au moins une huile volatile à température ambiante dans un rouge à lèvres ou fond de teint anhydre contenant des pigments et/ou des charges afin de diminuer le transfert du rouge à lèvres ou fond de teint.

17. Procédé cosmétique pour Imiter et/ou empêcher le transfert d'une composition de maquillage ou de soin cosmétique de la peau ou des lèvres sur un substrat différent de la dite peau et desdites lèvres, consistant à introduire dans la composition un organopolysiloxane élastomérique solide et une phase grasse renfermant au moins une huile volatile à température ambiante.

## Claims

1. Use of a solid, at least partially crosslinked, elastomeric organopolysiloxane combined with a fatty phase containing at least one oil which is volatile at room temperature, in a cosmetic composition or for manufacturing a dermatological composition in order to reduce the transfer of the said composition.

2. Use according to Claim 1, characterized in that the volatile oil is present in a proportion of 1 to 50% by weight, preferably 30 to 50%, of the total weight of the composition.

3. Use according to one of the preceding claims characterized in that the volatile oil is chosen from silicone oils containing a linear silicone structure and units with a pendant alkyl chain and/or an alkyl chain at the end of the silicone structure, these alkyl chains being linear or branched and containing from 3 to 10 carbon atoms; polydimethylsiloxanes with a linear chain having from 2 to 6 silicon atoms; cyclic silicones having from 3 to 7 units -R₁R₂SiO-, with R₁ and R₂ independently representing H, methyl, ethyl or phenyl; C₃ to C₂₀ isoparaffins, and mixtures thereof.

4. Use according to one of the preceding claims, characterized in that the elastomeric organopolysiloxane is obtained by addition reaction and crosslinking, in the presence of a catalyst, of at least:
(a) an organopolysiloxane having at least two C₂-C₆ lower alkenyl groups per molecule; and
(b) an organopolysiloxane having at least two hydrogen atoms linked to a silicon atom per molecule.

5. Use according to any one of the preceding claims, characterized in that the organopolysiloxane is chosen from:
- i) organopolysiloxanes comprising R₂SiO and RSiO_{1.5} units and optionally R₃SiO_{0.5} and/or SiO₂ units in which the radicals R, independently of each other, represent a hydrogen, an alkyl, an aryl, an unsaturated aliphatic group, the weight ratio of the units R₂SiO to the units RSiO_{1.5} ranging from 1/1 to 30/1;
- ii) organopolysiloxanes which are insoluble and swellable in silicone oil, obtained by addition of an organohydrogenopolysiloxane (1) and of an organopolysiloxane (2) having unsaturated aliphatic groups such that the amount of hydrogen or of unsaturated aliphatic groups in (1) and (2) respectively is between 1 and 20 mol% when the organopolysiloxane is non-cyclic and between 1 and 50 mol% when the organopolysiloxane is cyclic.

6. Use according to one of the preceding claims, characterized in that the fatty phase also contains at least one non-volatile oil.

7. Use according to one of the preceding claims, characterized in that the solid elastomeric organopolysiloxane is present in the elastomeric organopolysiloxane/fatty phase mixture in order to form a homogeneous gel in a concentration ranging from 0.3 to 40% by weight.

8. Use according to any one of the preceding claims, characterized in that the fatty phase also contains at least one non-volatile oil chosen from hydrocarbon oils of animal or plant origin; oils of formula R₉COOR₁₀ in which R₉ represents a higher fatty acid residue containing from 7 to 19 carbon atoms and R₁₀ represents a branched hydrocarbon chain containing from 3 to 20 carbon atoms; linear or branched hydrocarbons of mineral or synthetic origin; fatty alcohols; synthetic esters and ethers; linear polymethylsiloxanes which are liquid or pasty at room temperature; phenyldimethicones; phenyltrimethicones; polymethylphenylsiloxanes; fluoro oils which are optionally partially hydrocarbonated and/or siliconated; and mixtures thereof.

9. Use according to any one of the preceding claims, characterized in that it also contains at least one wax.

10. Use according to the preceding claim, characterized in that the wax is chosen from hydrocarbon, fluoro and silicone waxes and mixtures thereof.

11. Use according to Claim 9 or 10, characterized in that the wax is present in a proportion of 0 to 50% of the total weight of the composition, preferably 10 to 30%.

12. Use according to one of the preceding claims, characterized in that the composition also comprises a particulate phase present in a proportion of 0 to 35% of the total weight of the composition, preferably 5 to 25%.

13. Use according to one of the preceding claims, characterized in that the composition is in the form of a stick or tube, of supple or cast paste, or of a cream or a gel.

14. Use according to one of the preceding claims, characterized in that the composition also contains at least one cosmetic or dermatological active agent.

15. Use according to one of the preceding claims, characterized in that the composition is in the form of a foundation, a blusher, an eyeshadow, a concealer product, or a lipstick, in a care base or a fixing base for the lips, a skincare product or an antisun composition.

16. Use of at least one solid, at least partially crosslinked, elastomeric organopolysiloxane combined with a fatty phase containing at least one oil which is volatile at room temperature, in an anhydrous foundation or lipstick containing pigments and/or fillers, in order to reduce the transfer of the lipstick or foundation.

17. Cosmetic process for limiting and/or preventing the transfer of a make-up or cosmetic care composition for the skin or the lips onto a substrate other than the said skin and the said lips, this process consisting in introducing a solid elastomeric organopolysiloxane and a fatty phase containing at least one oil which is volatile at room temperature into the composition.

## Patentansprüche

1. Verwendung eines zumindest teilweise vernetzten, elastomeren, festen Organopolysiloxans in Kombination mit einer Fettphase, die mindestens ein bei Raumtemperatur flüchtiges Öl enthält, in einer kosmetischen Zusammensetzung oder zur Herstellung einer dermatologischen Zusammensetzung, um den Transfer der Zusammensetzung zu vermindern.

2. Verwendung nach Anspruch 1, dadurch gekennzeichnet, daß das flüchtige Öl in einen Mengenanteil im Bereich von 1 bis 50 Gew.-% und vorzugsweise im Bereich von 30 bis 50 % des Gesamtgewichts der Zusammensetzung vorliegt.

3. Verwendung nach einem der vorhergehenden Ansprüche, dadurch gekennzeichnet, daß das flüchtige Öl ausgewählt ist unter: den Siliconölen, die eine lineare Siliconstruktur und Einheiten mit Alkylkette als Seitenkette und/oder am Ende der Siliconstruktur aufweisen, wobei die Alkylketten geradkettig oder verzweigt vorliegen und 3 bis 10 Kohlenstoffatome enthalten; Polydimethylsiloxanen mit linearer Kette mit 2 bis 6 Siliciumatomen; cyclischen Siliconen mit 3 bis 7 Einheiten -R₁R₂SiO-, wobei R₁ und R₂ unabhängig voneinander H, Methyl, Ethyl oder Phenyl bedeuten; C₃₋₂₀-Isoparaffinen und deren Gemischen.

4. Verwendung nach einem der vorhergehenden Ansprüche, dadurch gekennzeichnet, daß das elastomere Organopolysiloxan in Gegenwart eines Katalysators durch Addition und Vernetzung zumindest der folgenden Verbindungen hergestellt wird:
(a) einem Organopolysiloxan mit mindestens zwei niederen C₂₋₆-Alkenylgruppen pro Molekül und
(b) einem Organopolysiloxan mit mindestens zwei, an ein Siliciumatom gebundenen Wasserstoffatomen pro Molekül.

5. Verwendung nach einem der vorhergehenden Ansprüche, dadurch gekennzeichnet, daß das Organopolysiloxan ausgewählt ist unter:
(i) den Organopolysiloxanen mit R₂SiO- und -RSiO₁,₅-Einheiten und gegebenenfalls R₃SiO_{0,5}- und/oder SiO₂-Einheiten, wobei die Gruppen R unabhängig voneinander Wasserstoff, Alkyl, Aryl oder eine ungesättigte aliphatische Gruppe bedeuten, wobei das Gewichtsverhältnis von R₂SiO-Einheiten zu RSiO_{1,5}-Einheiten im Bereich von 1/1 bis 30/1 liegt;
(ii) den Organopolysiloxanen, die in Siliconöl unlöslich und quellbar sind und die durch Addition eines Organohydrogenpolysiloxans (1) und eines Organopolysiloxans mit ungesättigten aliphatischen Gruppen (2) hergestellt werden, die so ausgewählt sind, daß der Mengenanteil von Wasserstoff oder ungesättigten aliphatischen Gruppen in (1) bzw. (2) im Bereich von 1 bis 20 Mol-%, wenn das Organopolysiloxan nicht cyclisch vorliegt, und im Bereich von 1 bis 50 Mol-% liegt, wenn das Organopolysiloxan cyclisch ist.

6. Verwendung nach einem der vorhergehenden Ansprüche, dadurch gekennzeichnet, daß die Fettphase ferner mindestens ein nichtflüchtiges Öl enthält.

7. Verwendung nach einem der vorhergehenden Ansprüche, dadurch gekennzeichnet, daß das elastomere, feste Organopolysiloxan in dem Gemisch elastomeres Organopolysiloxan/Fettphase zur Bildung eines homogenen Gels in einer Konzentration im Bereich von 0,3 bis 40 Gew.-% vorliegt.

8. Verwendung nach einem der vorhergehenden Ansprüche, dadurch gekennzeichnet, daß die Fettphase ferner mindestens ein nichtflüchtiges Öl enthält, das ausgewählt ist unter: den Kohlenwasserstoffölen tierischen oder pflanzlichen Ursprungs; den Ölen der Formel R₉COOR₁₀, worin R₉ den Rest einer höheren Fettsäure mit 7 bis 19 Kohlenstoffatomen und R₁₀ eine verzweigte Kohlenwasserstoffkette mit 3 bis 20 Kohlenstoffatomen bedeutet; den geradkettigen oder verzweigten Kohlenwasserstoffen mineralischen oder synthetischen Ursprungs; den Fettalkoholen; den synthetischen Estern und Ethern; den bei Raumtemperatur flüssigen oder pastösen, geradkettigen Polymethylsiloxanen; den Phenyldimethiconen; den Phenyltrimethiconen; den Polymethylphenylsiloxanen; den fluorierten Ölen, die gegebenenfalls zum Teil kohlenwasserstoff- und/oder siliconhaltig sind; und deren Gemischen.

9. Verwendung nach einem der vorhergehenden Ansprüche, dadurch gekennzeichnet, daß sie ferner mindestens ein Wachs enthält.

10. Verwendung nach dem vorhergehenden Anspruch, dadurch gekennzeichnet, daß das Wachs unter den Kohlenwasserstoffwachsen, den fluorierten Wachsen, den siliconhaltigen Wachsen und deren Gemischen ausgewählt ist.

11. Verwendung nach Anspruch 9 oder 10, dadurch gekennzeichnet, daß das Wachs in einem Mengenanteil im Bereich von 0 bis 50 % des Gesamtgewichts der Zusammensetzung und vorzugsweise im Bereich von 10 bis 30 % vorliegt.

12. Verwendung nach einem der vorhergehenden Ansprüche, dadurch gekennzeichnet, daß die Zusammensetzung ferner eine Partikelphase enthält, die in einem Mengenanteil von 0 bis 35 % des Gesamtgewichts der Zusammensetzung und vorzugsweise im Bereich von 5 bis 25 % vorliegt.

13. Verwendung nach einem der vorhergehenden Ansprüche, dadurch gekennzeichnet, daß die Zusammensetzung als Stick oder Stift, als weiche oder gegossene Paste oder als Creme oder Gel vorliegt.

14. Verwendung nach einem der vorhergehenden Ansprüche, dadurch gekennzeichnet, daß die Zusammensetzung ferner mindestens einen kosmetischen oder dermatologischen Wirkstoff enthält.

15. Verwendung nach einem der vorhergehenden Ansprüche, dadurch gekennzeichnet, daß die Zusammensetzung in Form einer Zusammensetzung für Make-up, als Wangenrouge, Lidschatten, Abdeckprodukt oder Lippenstift, in einer Pflegebasis oder fixierenden Masse für die Lippen, als Produkt zur Pflege der Haut oder als Sonnenschutzzusammensetzung vorliegt.

16. Verwendung mindestens eines zumindest zum Teil vernetzten, elastomeren, festen Organopolysiloxans in Kombination mit einer Fettphase, die mindestens ein bei Raumtemperatur flüchtiges Öl enthält, in einem wasserfreien Lippenstift oder Make-up, die Pigmente und/oder Füllstoffe enthalten, um den Transfer des Lippenstifts oder Make-ups zu vermindern.

17. Kosmetisches Verfahren, um den Transfer einer kosmetischen Zusammensetzung zum Schminken oder zur Pflege der Haut oder der Lippen auf ein Substrat, das von der Haut oder den Lippen verschieden ist, zu begrenzen und/oder zu verhindern, das darin besteht, in die Zusammensetzung ein festes, elastomeres Organopolysiloxan und eine Fettphase, die mindestens ein bei Raumtemperatur flüchtiges Öl enthält, einzuarbeiten.
